# EUROPÄISCHE PATENTSCHRIFT

(11) **EP 1 754 448 B1**
(45) Veröffentlichungstag und Bekanntmachung des Hinweises auf die Patenterteilung: **26.03.2008**
(21) Anmeldenummer: 05017752.6
(22) Anmeldetag: 16.08.2005
(51) Int. Cl.: A61B 19/00, B25J 9/16, B25J 19/00

(54) **Anthropomorpher medizintechnischer Roboterarm mit Bewegungseinschränkung**
Anthropomorphic medical robot arm with limitation of movement
Bras de robot médical anthropomorphe avec de limitation de mouvement

(43) Veröffentlichungstag der Anmeldung: 21.02.2007
(73) Patentinhaber: BrainLAB AG, 85551 Kirchheim/Heimstetten (DE); Deutsches Zentrum für Luft- und Raumfahrt e.V., 51147 Köln (DE)
(72) Erfinder: Birkenbach, Rainer, 85435 Erding (DE); Hartlep, Dr. Andreas, 83629 Naring (DE); Wohlgemuth, Richard, 83646 Bad Tölz (DE); Bertram, Michael, 85570 Markt Schwaben (DE); Albu-Schäfer, Alin, 81241 München (DE); Grebenstein, Markus, 80539 München (DE); Hagn, Ulrich, 82396 Pähl (DE); Jöhl, Klaus, 82205 Gilching (DE); Nickl, Mathias, 80333 München (DE); Ortmaier, Tobias, 82110 Germering (DE); Hacker, Franz, 86925 Fuchstal (DE); Jörg, Stefan, 81241 München (DE); Konietschke, Rainer, 82205 Gilching (DE)
(74) Vertreter: Schwabe, Hans-Georg

(56) Entgegenhaltungen:
- US-A- 4 046 262
- US-A- 4 828 453
- US-A- 4 911 033
- US-A- 4 986 723
- US-A- 5 791 231
- US-B1- 6 451 027
- US-B1- 6 642 686

## Beschreibung

Die vorliegende Erfindung betrifft einen anthropomorphen medizintechnischen Roboterarm sowie ein Verfahren zum Betrieb eines solchen Roboterarms. Anthropomorphe medizintechnische Roboterarme dienen beispielsweise der Unterstützung bei bestimmten Tätigkeiten im medizinischen Umfeld. Sie können verwendet werden, um chirurgische Werkzeuge wie Endoskope, Biopsienadeln, Bohrer oder Implantate zu positionieren oder zu manipulieren. Existierende Robotersysteme werden entweder ferngesteuert (beispielsweise durch eine Fernsteuerungs-Eingabevorrichtung) oder direkt manipuliert und arbeiten dabei teilweise oder vollständig autonom.

Die Firma Barrett Technology Inc, Cambridge, MA, USA bietet unter dem Namen "WAM" einen Roboterarm zur Verwendung in der bildgeführten Chirurgie und für viele andere Anwendungen an. Dieser mobile Arm mit einer kinematischen Kette bestehend aus einem Schultergelenk, einem Ellbogengelenk und einem Handgelenk stellt eine haptische Interaktion mit dem Verwender zur Verfügung und kann deshalb manuell geführt werden (siehe auch www.barretttechnology.com).

Die US-A-4 911 033, von der der Oberbegriff des Anspruchs 1 abgeleitet wird, offenbart einen anthropomorphen medizintechnischen Roboterarm mit einem proximalen Basisende und einem distalen Funktionsende. Der Arm weist ein Basisgelenk, ein Mittelgelenk und ein Distalgelenk auf, sowie ein erstes Armelement zwischen Basisgelenk und Mittelgelenk und ein zweites Armelement zwischen Mittelgelenk und Distalgelenk.

Die JP9-190207 schlägt vor, den Bewegungsbereich der Gelenkwinkel eines Robotmanipulators einzuschränken, um eine gegenseitige Behinderung der Teile des Armes zu vermeiden. Er wird also nur soweit eingeschränkt, dass der gesamte funktionell mögliche Arbeitsbereich des Roboterarms verwendet werden kann, jedoch Bewegungen außerhalb dieses funktionell möglichen Bewegungsbereiches vermieden werden.

Die EP 1 296 609 B1 beschreibt eine medizinische Positionierungsvorrichtung mit mindestens drei Gliedern, wobei die Gelenkachsen abfolgender Gelenke senkrecht ausgerichtet und so optimiert sind, dass das angebrachte chirurgische Werkzeug jedwedes Ziel am Patienten aus fast jeder Richtung erreichen kann.

Die US 2002/0050183 A1 beschreibt einen anthropomorphen Roboterarm, der an einem Torso befestigt ist, mit einer Struktur, welche das Auftreten eines Zustandes minimiert, wo eine geringe Veränderung des Ellbogens eine große Veränderung der Schulterposition notwendig macht.

Die US 2001/0013764 A1offenbart einen Manipulator zum Ausrichten eines chirurgischen Instruments mit einer Behandlungsstelle, und zwar durch eine Hand-Eingabesteuerung oder manuell.

Aus der WO 2003/077101 A3 ist ein Verfahren bekannt, bei dem für den Verwender eines chirurgischen Roboters ein haptisches Feedback beziehungsweise eine haptische Rückkopplung zur Verfügung gestellt wird und der Arm in Reaktion auf eine aufgebrachte Kraft positioniert wird. Durch dieses Verfahren wird die Bewegung des Armes durch eine Benutzerführung auf definierte Trajektorien oder auf Sicherheitszonen beschränkt.

Die Veröffentlichung "Entwicklung eines generischen, kraftreflektierenden Handkontrollers für unterschiedliche Szenarien der Medizintechnik" (U. Hagn, Projektberichte im Jahresbericht 2003 des bayrischen Kompetenznetzwerks für Mechatronik) beschreibt einen chirurgischen Robotmanipulator, welcher durch den Verwender geführt werden kann, wobei Manipulationskräfte gemessen und Gegenkräfte aufgebracht werden.

Die Veröffentlichung "Design requirements for a new robot for minimally invasive surgery (T. Ortmaier, H. Weiss and V. Falk in: Industrial Robotics: An International Journal, Special Edition on Medical Robotics. 31 (6), pp. 493-498, November 2004) beschreibt einen kinematisch redundanten, chirurgischen Roboterarm mit einer Kinematik und einer Verbindungslänge, die für benötigte Arbeitsräume mit unterschiedlichen minimal invasiven chirurgischen Tätigkeiten und bezüglich der Genauigkeit optimiert sind.

Es ist die Aufgabe der vorliegenden Erfindung, einen Roboterarm und ein Betriebsverfahren für einen Roboterarm zur Verfügung zu stellen, welche den Umgang des Verwenders mit dem Roboterarm vereinfachen beziehungsweise erleichtern.

Diese Aufgabe wird durch einen anthropomorphen medizinischen Roboterarm gemäß dem Anspruch 1, durch ein Verfahren zum Betrieb eines anthropomorphen medizintechnischen Roboterarms gemäß dem Anspruch 9, durch ein Programm gemäß dem Anspruch 18 sowie ein Computerprogramm - Speichermedium gemäß dem Anspruch 19 gelöst. Die Unteransprüche definieren bevorzugte Ausführungsformen der Erfindung.

Gemäß der vorliegenden Erfindung sind bei dem Roboterarm auf die Gelenke wirkende Bewegungshemmer vorgesehen, welche den funktionellen Arbeitsbereich des Roboterarms durch eine Hemmung der Bewegungsfreiheit der Gelenke auf den möglichen Bewegungsbereich eines menschlichen Arms einschränken. Dabei arbeitet das Basisgelenk im wesentlichen wie ein Kugelgelenk mit der Bewegungsfreiheit eines menschlichen Schultergelenks, das Mittelgelenk arbeitet im wesentlichen wie Scharniere mit der Bewegungsfreiheit ähnlich einem menschlichen Ellbogen und das Distalgelenk arbeitet wieder im wesentlichen wie zwei orthogonale Scharniere mit der Bewegungsfreiheit ähnlich einem menschlichen Handgelenks (Ellenbogen und Handgelenk sind gleich aufgebaut als Knick-Roll-Gelenke). Mit anderen Worten kann der Roboterarm gemäß der vorliegenden Erfindung in seiner Bewegungsfreiheit so eingeschränkt werden, dass er sich wie ein menschlicher Arm bewegt, also nicht mehr seinen gesamten funktionell möglichen Bewegungsbereich benutzt.

Wenn hierin der Ausdruck" funktionell möglicher Bewegungsbereich" verwendet wird, so meint dieser den Bewegungsbereich, der sinnvoll durch den Roboterarm verwendet werden könnte, ohne dass der Arm sich selbst im Weg steht, behindert oder Glieder aneinander stoßen. Diesen funktionell bzw. sinnvollerweise möglichen, gesamten Arbeitsbereich des Roboterarms schränkt die vorliegende Erfindung noch auf den möglichen Bewegungsbereich eines menschlichen Armes ein, und es entsteht der große Vorteil, dass die Bewegungen eines solchermaßen gesteuerten oder per Hand geführten Armes für den Verwender vorhersehbar und intuitiv erfassbar werden.

Im Gegensatz hierzu gestatten existierende Roboterarme, die den gesamten funktionell möglichen und sinnvollen Arbeitsbereich ausnutzen, Bewegungen, die nicht intuitiv erfassbar oder vorhersagbar sind. Dies kann einerseits dazu führen, dass der Verwender durch die Bewegung des Roboters überrascht wird und im schlimmsten Fall der Roboter mit Behandlungspersonal oder Behandlungsvorrichtungen kollidiert, ohne dass dies vorhersagbar und abwendbar gewesen wäre. Gemäß der Erfindung wird aber ein Verwender intuitiv wissen, welche Bewegung der Roboter als nächstes ausführen könnte da er solche Bewegungen von seinem eigenen Arm her kennt. Durch die verbesserte intuitive Verwendung wird die Lernzeit für die Betätigung der Vorrichtung abgekürzt und die Sicherheit erhöht. Die Bewegungseinschränkungen gestatten es, ein chirurgisches Umfeld aufzubauen wo der Raum begrenzt ist, und zwar in ähnlicher Weise oder sogar in gleicher Weise wie bei einem Aufbau für eine übliche per Hand durchgeführte Prozedur. Trotz der Bewegungseinschränkungen ist die Kinematik des Arms noch eine solche, dass sie für alle möglichen chirurgischen Anwendungen geeignet ist, ebenso wie beim menschlichen Arm.

Anders formuliert erzielt die Erfindung ihre Vorteile durch die anthropomorphe Ausgestaltung des Armes, der durch den Verwender geführt wird, und zwar durch Anfassen des Armes und damit verbundenem Aufbringen von Kräften und Momenten (haptische Interaktion) in Verbindung mit bestimmten Bewegungseinschränkungen des Armes. Zusätzlich kann der Arm gleichzeitig als haptische Rückkopplungsvorrichtung verwendet werden und dabei für den Verwender Informationen liefern. Der Roboterarm stellt vorteilhafterweise ebenso wie der menschliche Arm, mindestens einen redundanten Bewegungsfreiheitsgrad zur Verfügung. Um die intuitive Verwendung zu verbessern und die Voraussage von Bewegungen des ersten Armgliedes und des zweiten Armgliedes zu erleichtern, können die Bewegungen weiter eingeschränkt werden. Die intuitivste Bewegungseinschränkung ist die, die dem menschlichen Arm vergleichbar ist, aber ebenso wie eine Anatomie variiert kann auch ein typischer (Tolleranz-) Bereich für die Einschränkung gewählt werden oder der Bereich verschiedener individueller Anatomien Anwendung finden.

Der Roboterarm kann Positions- und/oder Winkel- und/oder Drehmomentsensoren an den Gelenken aufweisen, die mit den Bewegungshemmern oder deren Steuerungen verbunden sind. Er kann mindestens sieben Gelenkverbindungen aufweisen, wobei mindestens drei im Basisgelenk, mindestens zwei im Mittelgelenk und mindestens zwei im Distalgelenk gruppiert sind. Am distalen Funktionsende, insbesondere hinter dem Distalgelenk, kann der Roboterarm einen Werkzeug- bzw. Instrumentenadapter aufweisen.

Die Bewegungshemmer an oder in den Gelenken können Bremsen oder Antriebseinrichtungen umfassen. Solche Antriebseinrichtungen können beispielsweise Elektromotoren sein, aber auch Kraftübertragungseinrichtungen wie Riemen und Zahnräder. Durch die Bremsen oder die Antriebseinrichtungen können haptische Rückkopplungen wie Gegenkräfte aufgebaut werden. Ein Zu- und/oder Abschalter für die Bewegungshemmer kann bereitgestellt werden, und dieser kann insbesondere eine der folgenden Vorrichtungen umfassen:
- einen mechanischen Schalter für mindestens eines der Gelenke;
- einen softwaregesteuerten Schalter für mindestens eines der Gelenke;
- einen kabelgesteuerten oder kabellos angesteuerten Schalter für mindestens eines der Gelenke.

Durch das Bereitstellen eines Zu- oder Abschalters für die Bewegungshemmer, insbesondere durch die Bereitstellung eines Schalters für das Mittelgelenk kann beispielsweise eine oder mehrere der Gelenkverbindungen gelöst, also eine Bewegungseinschränkung weggenommen werden. Weil dabei aber ein Schalter an dem Gelenk gedrückt werden muss (dies kann für alle Gelenke gelten) geschieht die Aufhebung der Bewegungseinschränkung nur unter voller Kontrolle des Verwenders.

Der Arm kann gemäß einer Ausführungsform eine Signalvorrichtung umfassen oder mit einer Signalvorrichtung verbunden sein, die bei einer unerlaubten Abweichung ein sichtbares, ein spürbares oder ein hörbares Signal abgibt. Auch besteht die Möglichkeit, an dem Arm mindestens eine Markeranordnung anzuordnen, deren Position und Lage von einem Tracking- bzw. Navigationssystem geortet und verfolgt werden kann.

Bei dem erfindungsgemäßen Verfahren wird der funktionell mögliche Arbeitsbereich des Roboterarmes durch eine Hemmung der Bewegungsfreiheit der Gelenke auf den möglichen Bewegungsbereich eines menschlichen Armes eingeschränkt. Die entsprechenden Vorteile wurden oben schon beschrieben. Die verfahrensmäßige Umsetzung der oben beschriebenen Merkmale ist möglich. Wenn beispielsweise eine am Arm angeordnete Markeranordnung von einem medizinischen Tracking- bzw. Navigationssytem positioniert geortet und verfolgt wird, kann dabei der Arm, insbesondere redundant, lokalisiert werden.

Die Armbewegung kann, wie oben schon angesprochen, durch das Eliminieren eines redundanten Bewegungsfreiheitsgrades gesteuert werden, und zwar insbesondere unter mindestens einer der folgenden Vorraussetzungen:
- der vertikale und/oder horizontale Abstand des Mittelgelenks zum proximalen Ende wird minimiert oder maximiert;
- die Haltung des Arms wird energetisch optimiert; und
- der Abstand des Mittelgelenks zum proximalen Ende oder zu einem Punkt in der Umgebung bleibt konstant.

Gemäß einer bevorzugten Ausführungsform wird die Einschränkung des möglichen Bewegungsbereichs oder der Übergang zu einer Einschränkung ausgelöst durch einen mittels Software erfassten Status, durch eine Haltung des Arms, durch eine Benutzereingabe oder durch eine vom Armsteuerungssystem selbst erzeugte Interaktion bzw. Aktion.

Die Erfindung wird im Weiteren anhand eine bevorzugten Ausführungsform näher erläutert. Sie kann alle hierin beschriebenen Merkmale einzeln oder in jedweder Kombination umfassen. In den beigelegten Zeichnungen zeigen:
- Figuren 1 und 2:: einen erfindungsgemäßen Roboterarm in unterschiedlichen Betätigungszuständen;
- Figur 3:: eine schematische Zeichnung für den Roboterarm-Aufbau.

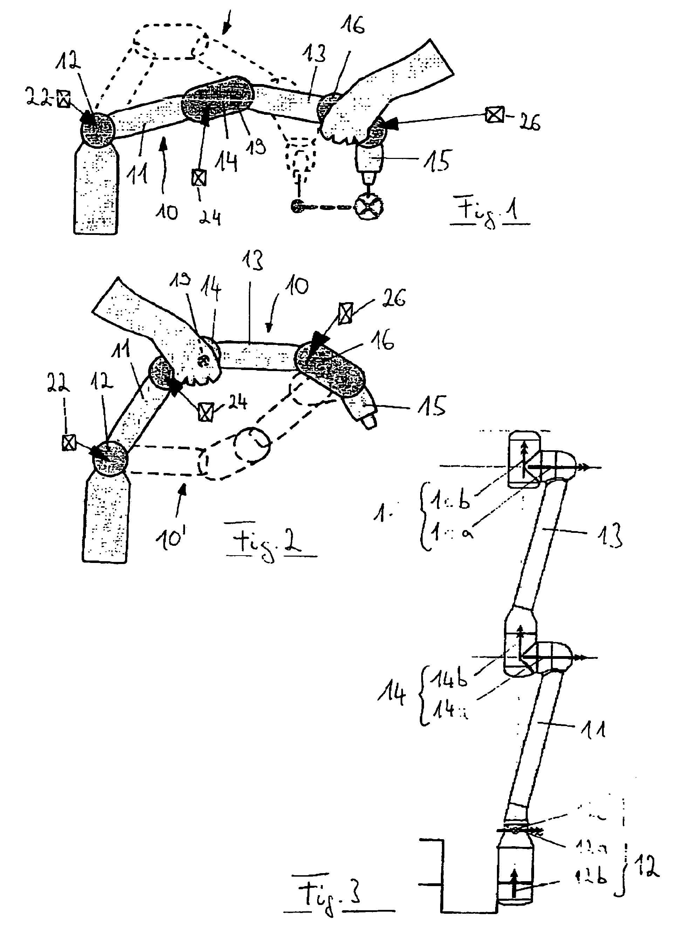

In den Figuren 1 und 2 ist schematisch ein erfindungsgemäßer Roboterarm dargestellt. Der Arm ist mit dem Bezugszeichen 10 versehen, und er sitzt auf einer nicht gekennzeichneten Basis, auf der sich das erste zum Arm gehörende Gelenk 12 befindet, das im weiteren auch Basisgelenk oder Schultergelenk genannt wird. Wie aus der Schemazeichnung in Figur 2 hervorgeht, weist das Basisgelenk 12 mehrere gelenkige Verbindungen oder Gelenkverbindungen auf, die als Drehachsen aufgezeigt sind. Der Figur 3 ist unten zu entnehmen, dass das Basis- oder Schultergelenk 12 drei Achsen oder Gelenkverbindungen aufweist, die durch Pfeile dargestellt sind, wobei der Pfeil 12c senkrecht zur Zeichenebene liegt.

Über dem Schultergelenk 12 ist das erste Armelement 11 gelenkig angebracht, das auch als "Oberarm" des Roboterarms 10 bezeichnet werden könnte. Es geht in Richtung des distalen Endes bis zum Gelenk 14, das zwei Gelenkverbindungen bzw. zwei Drehachsen aufweist. Diese Drehachsen sind in Figur 3 wiederum durch Pfeile dargestellt, die mit den Bezugszeichen 14a und 14 b versehen worden sind. Das Mittelgelenk 14 könnte auch als "Ellbogengelenk" oder "Ellbogen" bezeichnet werden. Vom Ellbogen 14 aus erstreckt sich das zweite Armelement 13 (oder der "Unterarm") zum Distalgelenk 16, das auch als "Handgelenk" bezeichnet werden kann. Das Handgelenk 16 hat, wie wiederum aus Figur 3 hervorgeht, auch zwei Drehachsen 16a, 16b, entsprechend den Drehachsen der Gelenkverbindungen 14a, 14b des Ellbogengelenkes 14.

Am Handgelenk 16 vorne ist dann ein nicht explizit dargestellter Flansch angebracht, über den ein Werkzeug befestigt werden kann. In Figur 1 und Figur 2 ist als Werkzeug beispielsweise der Bohrer 15 gezeigt.

Es werden leicht bauende Materialien verwendet, und es entsteht somit ein anthropomorpher Roboterarm 10 mit mindestens sieben Gelenkverbindungen bzw. Drehachsen, von denen mindestens drei in der Schulter 12, mindestens zwei im Ellbogen 14 und mindestens zwei im Handgelenk 16 gruppiert sind.

Die Gelenke 12, 14 und 16 und insbesondere auch ihre Einzel-Gelenkverbindungen 12 a-c, 14a,b und 16a,b sind mit Drehmomentsensoren und Positionssensoren versehen. Über diese Sensoren, die nicht explizit dargestellt sind, können die Winkelstellung und die Drehmomentverhältnisse in den einzelnen Gelenken erfasst werden, und dadurch grundsätzlich auch die Position und Orientierung der Werkzeugspitze zu jedem Zeitpunkt. Gegebenenfalls muss der Roboterarm vor dem Einsatz hierzu kalibriert werden (Nullstellung).

Mit den Gelenken verbunden oder an den Gelenken angebracht, in jedem Fall jedoch auf die Gelenke wirkend sind die Bewegungshemmer 22, 24, 26 für Schultergelenk 12, Ellbogengelenk 14 und Handgelenk 16 vorgesehen. Diese Bewegungshemmer könnten beispielsweise Bremsen oder Antriebseinrichtungen (Servomotoren, Riemen, Zahnräder) sein und sie sind zu- und abschaltbar. Die einzelnen Bewegungshemmer 22, 24 und 26 können nicht nur auf die Gelenke 12, 14 und 16 wirken, sondern auch auf deren Einzel-Gelenkverbindungen oder Drehachsen (a, b, c).

Die Bewegungshemmer 22, 24 und 26 sind insgesamt oder einzeln zu - und/oder abschaltbar, und sie werden so gesteuert, das der Arm den möglichen Bewegungsbereich eines menschlichen Arms erhält. Dabei können durchaus gewisse Tolleranzbereiche aufgebaut und zugelassen werden, wie dies bei menschlichen Armen ebenfalls der Fall ist. Der Arm 10 kann auch vorteilhafterweise insgesamt Abmessungen haben, die denjenigen eines menschlichen Armes in etwa gleichen oder sich in einem Bereich von tatsächlich möglichen menschlichen Armgrößen befinden. Mit den Bewegungshemmern 22, 24 und 26 ergibt sich nun eine gewisse Bewegungsfreiheit, bei welcher der Arm ohne weiteres durch ein Angreifen des Benutzers (dargestellte Hand) bewegt werden kann (haptische Interaktion). Eine solche Bewegung ist beispielsweise beim Übergang aus der gestrichelten Position 10' in die Position 10 in Figur 1 dargestellt.

Für den Übergang von der Position 10' (gestrichelt) zur Position 10 in Figur 2 kommt es möglicherweise zu Bewegungen, die nicht durch den menschlichen Arm nachvollzogen werden könnten. Der Arm würde dann dieser Bewegung mit Hilfe der Bewegungshemmer 22, 24 und 26 einen Widerstand entgegensetzen bzw. die Bewegung nicht ohne weiteres gestatten (haptische Rückkopplung/Aufbau einer Gegenkraft).

In diesem Fall besteht die Möglichkeit, dass der Verwender den Abschaltknopf 19 betätigt, der in Figur 2 unter der Hand (gestrichelt) dargestellt ist. Der Benutzer führt damit eine Umgehung beziehungsweise einen "override" durch, indem er durch den Druck auf den Knopf 19 die Bewegung des Armes 10 willentlich freigibt. Wenn der Knopf wieder losgelassen wird, wird der Arm 10 sich wieder lediglich wie ein menschlicher Arm bewegen lassen. Der Knopf 19 kann hierbei auf eines oder mehrere Gelenke oder auf eine oder mehrere Gelenkverbindungen wirken.

Die Bereitstellung des erfindungsgemäßen Roboterarms und sein erfindungsgemäßer Betrieb mit den oben aufgezeigten Möglichkeiten stellt für den Benutzer ein intuitiv beherrschbares, im Verhalten vorraussagbares und leicht erlernbares Hilfsmittel zur Verfügung.

## Patentansprüche

1. Anthropomorpher medizintechnischer Roboterarm (10) mit einem proximalen Basisende und einem distalen Funktionsende, wobei der Arm (10) ein Basisgelenk (12), ein Mittelgelenk (14) und ein Distalgelenk (16) aufweist, sowie ein erstes Armelement (11) zwischen Basisgelenk (12) und Mittelgelenk (14) und ein zweites Armelement (13) zwischen Mittelgelenk und Distalgelenk (16), **dadurch gekennzeichnet, dass** zu- und/oder abschaltbare, auf die Gelenke (12, 14, 16) wirkende Bewegungshemmer (22, 24, 26) vorgesehen sind, welche den funktionell möglichen Arbeitsbereich des Roboterarms (10) durch eine Hemmung der Bewegungsfreiheit der Gelenke (12, 14, 16) auf den möglichen Bewegungsbereich eines menschlichen Arms einschränken.

2. Roboterarm nach Anspruch 1, **dadurch gekennzeichnet, dass** er Positions- und/oder Winkel- und/oder Drehmomentsensoren an den Gelenken aufweist, die mit den Bewegungshemmern oder deren Steuerungen verbunden sind.

3. Roboterarm nach Anspruch 1 oder 2, **dadurch gekennzeichnet, dass** er mindestens 7 Gelenkverbindungen aufweist, wobei davon drei im Basisgelenk (12), zwei im Mittelgelenk (14) und zwei im Distalgelenk (16) gruppiert sind.

4. Roboterarm nach einem der Ansprüche 1 bis 3, **dadurch gekennzeichnet, dass** er am distalen Funktionsende, insbesondere hinter dem Distalgelenk, einen Werkzeug- bzw. Instrumentenadapter aufweist.

5. Roboterarm nach einem der Ansprüche 1 bis 4, **dadurch gekennzeichnet, dass** die Bewegungshemmer an oder in den Gelenken (12, 14, 16) Bremsen oder Antriebseinrichtungen umfassen.

6. Roboterarm nach einem der Ansprüche 1 bis 5, **dadurch gekennzeichnet, dass** der Zu- und/oder Abschalter für die Bewegungshemmer mindestens eine der folgenden Vorrichtungen aufweist:
- einen mechanischen Schalter für mindestens eines der Gelenke (12, 14, 16);
- einen softwaregesteuerten Schalter für mindestens eines der Gelenke (12, 14, 16);
- einen kabelgesteuerten oder kabellos angesteuerten Schalter für mindestens eines der Gelenke (12, 14, 16).

7. Roboterarm nach einem der Ansprüche 1 bis 6, **dadurch gekennzeichnet, dass** er eine Signalvorrichtung umfasst, die bei einer unerlaubten Bewegungsabweichung ein sichtbares, ein spürbares oder ein hörbares Signal abgibt.

8. Roboterarm nach einem der Ansprüche 1 bis 7, **dadurch gekennzeichnet, dass** an ihm mindestens eine Markeranordnung angeordnet ist, die von einem medizintechnischen Tracking- bzw. Navigationssystem positionell geortet und verfolgt werden kann.

9. Verfahren zum Betrieb eines anthropomorphen medizintechnischen Roboterarms (10) mit einem proximalen Basisende und einem distalen Funktionsende, wobei der Arm (10) ein Basisgelenk (12), ein Mittelgelenk (14) und ein Distalgelenk (16) aufweist, sowie ein erstes Armelement (11) zwischen Basisgelenk (12) und Mittelgelenk (14) und ein zweites Armelement (13) zwischen Mittelgelenk und Distalgelenk (16), bei dem der funktionell mögliche Arbeitsbereich des Roboterarms (10) zuschaltbar bzw. abschaltbar durch eine Hemmung der Bewegungsfreiheit der Gelenke (12, 14, 16) auf den möglichen Bewegungsbereich eines menschlichen Arms eingeschränkt wird.

10. Verfahren nach Anspruch 9, bei dem Positionen und/oder Winkel und/oder Drehmomente an den Gelenken erfasst und bei der Bewegungseinschränkung oder der Gelenksteuerung berücksichtigt werden .

11. Verfahren nach Anspruch 9 oder 10, bei dem zur Bewegungshemmung an oder in den Gelenken (12, 14, 16) gebremst oder angetrieben wird.

12. Verfahren nach einem der Ansprüche 9 bis 11, bei dem die Zu- und/oder Abschaltung für die Bewegungshemmung über mindestens eine der folgenden Vorrichtungen erfolgt:
- einen mechanischen Schalter für mindestens eines der Gelenke (12, 14, 16);
- einen softwaregesteuerten Schalter für mindestens eines der Gelenke (12, 14, 16);
- einen kabelgesteuerten oder kabellos angesteuerten Schalter für mindestens eines der Gelenke (12, 14, 16).

13. Verfahren nach einem der Ansprüche 9 bis 12, bei dem der Arm (10) mindestens 7 Gelenkverbindungen aufweist, wobei davon drei im Basisgelenk (12), zwei im Mittelgelenk (14) und zwei im Distalgelenk (16) gruppiert sind, und bei dem eine Gelenkverbindung am Mittelgelenk (14) fixiert ist und durch eine Schaltung, insbesondere einen Knopfdruck gelöst werden kann.

14. Verfahren nach einem der Ansprüche 9 bis 13, bei dem bei einer unerlaubten oder unerwünschten Bewegungsabweichung durch eine Signalvorrichtung umfasst ein sichtbares, ein spürbares oder ein hörbares Signal abgegeben wird.

15. Verfahren nach einem der Ansprüche 9 bis 14, bei dem die Position und Orientierung mindestens einer am Arm (10) angeordneten Markeranordnung von einem Tracking- bzw. Navigationssystem geortet und verfolgt werden kann, um damit, insbesondere redundant, den Arm zu lokalisieren.

16. Verfahren nach einem der Ansprüche 9 bis 15, bei dem die Armbewegung gesteuert wird durch das Eliminieren eines redundanten Bewegungsfreiheitsgrades, und zwar unter mindestens einer der folgenden Voraussetzungen:
- der vertikale und/oder horizontale Abstand des Mittelgelenks (14) zum proximalen Ende wird minimiert oder maximiert;
- die Haltung des Arms ist energetisch optimiert; und
- der Abstand des Mittelgelenks (14) zum proximalen Ende oder zu einem Punkt in der Umgebung bleibt konstant.

17. Verfahren nach einem der Ansprüche 9 bis 16, bei dem die Einschränkung des möglichen Bewegungsbereichs oder der Übergang zu einer Einschränkung ausgelöst wird durch einen mittels Software erfassten Status, eine Haltung des Arms, eine Benutzereingabe oder eine vom Armsteuerungssystem selbst erzeugte Interaktion.

18. Programm, das, wenn es auf einem Computer läuft oder in einem Computer geladen ist, den Computer veranlasst, ein Verfahren gemäß einem der Ansprüche 9 bis 17 durchzuführen.

19. Computerprogramm-Speichermedium, das ein Programm nach Anspruch 18 aufweist.

## Claims

1. An anthropomorphic medical robot arm (10) comprising a proximal base end and a distal functional end, wherein the arm (10) comprises a base joint (12), a middle joint (14) and a distal joint (16), and comprising a first arm element (11) between the base joint (12) and the middle joint (14) and a second arm element (13) between the middle joint and the distal joint (16), **characterised in that** movement inhibitors (22, 24, 26) are provided which can be switched on and off and act on the joints (12, 14, 16) and which restrict the functionally possible working range of the robot arm (10) to the possible range of movement of a human arm by inhibiting the freedom of movement of the joints (12, 14, 16).

2. The robot arm according to claim 1, **characterised in that** it comprises position and/or angle and/or torque sensors on its joints, said sensors being connected to the movement inhibitors or their controls.

3. The robot arm according to claim 1 or 2, **characterised in that** it comprises at least seven joint connections, wherein three of them are grouped in the base joint (12), two in the middle joint (14), and two in the distal joint (16).

4. The robot arm according to any one of claims 1 to 3, **characterised in that** it comprises a tool or instrument adaptor at the distal functional end, in particular behind the distal joint.

5. The robot arm according to any one of claims 1 to 4, **characterised in that** the movement inhibitors on or in the joints (12, 14, 16) comprise brakes or drive means.

6. The robot arm according to any one of claims 1 to 5, **characterised in that** the on-switch and/or off-switch for the movement inhibitors comprises at least one of the following devices:
- a mechanical switch for at least one of the joints (12, 14, 16);
- a software-controlled switch for at least one of the joints (12, 14, 16);
- a wire-controlled or wirelessly controlled switch for at least one of the joints (12, 14, 16).

7. The robot arm according to any one of claims 1 to 6, **characterised in that** it comprises a signal device which emits a visible, tangible or audible signal in the event of a prohibited movement deviation.

8. The robot arm according to any one of claims 1 to 7, **characterised in that** at least one marker array is arranged on it, which can be positionally located and tracked by a medical tracking and/or navigation system.

9. A method for operating an anthropomorphic medical robot arm (10) comprising a proximal base end and a distal functional end, wherein the arm (10) comprises a base joint (12), a middle joint (14) and a distal joint (16), and comprising a first arm element (11) between the base joint (12) and the middle joint (14) and a second arm element (13) between the middle joint and the distal joint (16), wherein the functionally possible working range of the robot arm (10) is restricted, in a manner which can be switched on and/or off, to the possible range of movement of a human arm by inhibiting the freedom of movement of the joints (12, 14, 16).

10. The method according to claim 9, wherein positions and/or angles and/or torques on the joints are detected and taken into account when restricting the movement or controlling the joints.

11. The method according to claim 9 or 10, wherein movement is inhibited on or in the joints (12, 14, 16) by braking or driving.

12. The method according to any one of claims 9 to 11, wherein the inhibiting of the movement is switched on and/or off via at least one of the following devices:
- a mechanical switch for at least one of the joints (12, 14, 16);
- a software-controlled switch for at least one of the joints (12, 14, 16);
- a wire-controlled or wirelessly controlled switch for at least one of the joints (12, 14, 16).

13. The method according to any one of claims 9 to 12, wherein the arm (10) comprises at least seven joint connections, wherein three of them are grouped in the base joint (12), two in the middle joint (14), and two in the distal joint (16), and wherein one joint connection is fixed to the middle joint (14) and can be released by a switch, in particular by pressing a button.

14. The method according to any one of claims 9 to 13, wherein a visible, tangible or audible signal is emitted by a signal device in the event of a prohibited or undesired movement deviation.

15. The method according to any one of claims 9 to 14, wherein the position and orientation of at least one marker array arranged on the arm (10) can be located and tracked by a tracking and/or navigation system, so as to localise the arm, in particular redundantly.

16. The method according to any one of claims 9 to 15, wherein the movement of the arm is controlled by eliminating a redundant degree of freedom of movement, given at least one of the following conditions:
- the vertical and/or horizontal distance from the middle joint (14) to the proximal end is minimised or maximised;
- the holding of the arm is energetically optimised; and
- the distance from the middle joint (14) to the proximal end or to a point in the vicinity remains constant.

17. The method according to any one of claims 9 to 16, wherein a restriction of the possible range of movement or the transition to a restriction is triggered by: a status detected by means of software; holding the arm; a user input; or an interaction or action generated by the arm control system itself.

18. A program which, when it is running on a computer or is loaded onto a computer, causes the computer to perform a method in accordance with any one of claims 9 to 17.

19. A computer program storage medium comprising a program according to claim 18.

## Revendications

1. Bras de robot médical (10) anthropomorphe avec une extrémité de base proximale et une extrémité fonctionnelle distale, le bras (10) comportant une articulation de base (12), une articulation centrale (14) et une articulation distale (16), ainsi qu'un premier élément de bras (11) entre l'articulation de base (12) et l'articulation centrale (14) et un second élément de bras (13) entre l'articulation centrale et l'articulation distale (16), **caractérisé en ce que** sont prévus des inhibiteurs de mouvement (22, 24, 26) agissant sur les articulations (12, 14, 16) et pouvant être activés et/ou désactivés, lesquels limitent la plage de travail fonctionnellement possible du bras de robot (10), par un blocage de la liberté de mouvement des articulations (12, 14, 16), à la plage de mouvement possible d'un bras humain.

2. Bras de robot selon la revendication 1, **caractérisé en ce qu'**il comporte des capteurs de position et/ou angulaires et/ou de couple de rotation au droit des articulations, lesquels sont reliés aux inhibiteurs de mouvement ou à leurs commandes.

3. Bras de robot selon la revendication 1 ou 2, **caractérisé en ce qu'**il comporte au moins 7 liaisons articulées, trois étant regroupées dans l'articulation de base (12), deux dans l'articulation centrale (14) et deux dans l'articulation distale (16).

4. Bras de robot selon l'une quelconque des revendications 1 à 3, **caractérisé en ce qu'**il comporte un adaptateur d'outil ou adaptateur d'instrument à l'extrémité fonctionnelle distale, en particulier derrière l'articulation distale.

5. Bras de robot selon l'une quelconque des revendications 1 à 4, **caractérisé en ce que** les inhibiteurs de mouvement comprennent des freins ou des dispositifs d'entraînement sur ou dans les articulations (12, 14, 16).

6. Bras de robot selon l'une quelconque des revendications 1 à 5, **caractérisé en ce que** l'interrupteur de marche et/ou l'interrupteur d'arrêt pour les inhibiteurs de mouvement comportent au moins l'un des dispositifs suivants :
- un interrupteur mécanique pour au moins l'une des articulations (12, 14, 16) ;
- un interrupteur commandé par logiciel pour au moins l'une des articulations (12, 14, 16) ;
- un interrupteur commandé par câble ou sans câble pour au moins l'une des articulations (12, 14, 16).

7. Bras de robot selon l'une quelconque des revendications 1 à 6, **caractérisé en ce qu'**il comprend un dispositif de signalisation qui délivre un signal visible, un signal sensible ou un signal audible dans le cas d'un écart de mouvement non autorisé.

8. Bras de robot selon l'une quelconque des revendications 1 à 7, **caractérisé en ce qu'**il est disposé sur lui au moins un agencement de repères dont la position peut être localisée et suivie par un système médical de poursuite ou de navigation.

9. Procédé d'exploitation d'un bras de robot médical (10) anthropomorphe avec une extrémité de base proximale et une extrémité fonctionnelle distale, le bras (10) comportant une articulation de base (12), une articulation centrale (14) et une articulation distale (16), ainsi qu'un premier élément de bras (11) entre l'articulation de base (12) et l'articulation centrale (14) et un second élément de bras (13) entre l'articulation centrale et l'articulation distale (16), dans lequel la plage de travail fonctionnellement possible du bras de robot (10) est limitée à la plage de mouvement possible d'un bras humain, par activation ou désactivation, par un blocage de la liberté de mouvement des articulations (12, 14, 16).

10. Procédé selon la revendication 9, dans lequel des positions et/ou des angles et/ou des couples de rotation au droit des articulations sont relevés et pris en compte lors de la limitation du mouvement ou de la commande des articulations.

11. Procédé selon la revendication 9 ou 10, dans lequel on freine ou entraîne pour le blocage du mouvement, sur ou dans les articulations (12, 14, 16).

12. Procédé selon l'une quelconque des revendications 9 à 11, dans lequel l'activation et/ou la désactivation pour le blocage du mouvement s'effectuent par l'intermédiaire de l'un des dispositifs suivants :
- un interrupteur mécanique pour au moins l'une des articulations (12, 14, 16) ;
- un interrupteur commandé par logiciel pour au moins l'une des articulations (12, 14, 16) ;
- un interrupteur commandé par câble ou sans câble pour au moins l'une des articulations (12, 14, 16).

13. Procédé selon l'une quelconque des revendications 9 à 12, dans lequel le bras (10) comporte au moins 7 liaisons articulées dont trois sont regroupées dans l'articulation de base (12), deux dans l'articulation centrale (14) et deux dans l'articulation distale (16), et dans lequel une liaison articulée est fixée à l'articulation centrale (14) et peut être déverrouillée par un circuit, en particulier une pression sur un bouton.

14. Procédé selon l'une quelconque des revendications 9 à 13, dans lequel, en cas d'écart de mouvement non autorisé ou indésirable, un signal visible, un signal sensible ou un signal audible est délivré par un dispositif de signalisation.

15. Procédé selon l'une quelconque des revendications 9 à 14, dans lequel la position et l'orientation d'au moins un agencement de repères, disposé sur le bras (10), peuvent être localisées et suivies par un système de poursuite ou de navigation, afin de localiser ainsi le bras, en particulier de manière redondante.

16. Procédé selon l'une quelconque des revendications 9 à 15, dans lequel le mouvement du bras est commandé par l'élimination d'un degré de liberté de mouvement redondant, et à au moins l'une des conditions suivantes :
- la distance verticale et/ou horizontale de l'articulation centrale (14) par rapport à l'extrémité proximale est minimisée ou maximisée ;
- le maintien du bras est optimisé de manière énergétique ; et
- la distance de l'articulation centrale (14) à l'extrémité proximale ou à un point de l'environnement reste constante.

17. Procédé selon l'une quelconque des revendications 9 à 16, dans lequel la limitation de la plage de mouvement possible ou le passage à une limitation est déclenché par un statut, détecté au moyen du logiciel, un maintien du bras, une saisie d'un utilisateur ou une interaction produite par le système de commande de bras lui-même.

18. Programme qui, lorsqu'il tourne sur un ordinateur ou est chargé dans un ordinateur, amène l'ordinateur à mettre en oeuvre un procédé selon l'une des revendications 9 à 17.

19. Support de mémoire de programme d'ordinateur qui comporte un programme selon la revendication 18.
